# FASCICULE DE BREVET EUROPEEN

(11) **EP 2 921 172 B1**
(45) Date de publication et mention de la délivrance du brevet: **12.09.2018**
(21) Numéro de dépôt: 15160222.4
(22) Date de dépôt: 05.07.2007
(51) Int. Cl.: A61K 31/215, A61K 31/22, A61K 31/663, A61K 31/675, A61P 17/00, A61P 19/00, A61P 43/00

(54) **COMBINAISON D'UN INHIBITEUR DE HMG-COA RÉDUCTASE ET D'UN INHIBITEUR DE FARNÉSYL-PYROPHOSPHATASE SYNTHASE POUR TRAITER LES MALADIES LIÉES A L'ACCUMULATION ET/OU LA PERSISTANCE DE PROTÉINES PRÉNYLÉES**
Kombination eines HMG-CoA-reduktase-hemmers und eines farnesyl-pyrophosphatase-synthase-hemmers zur behandlung von erkrankungen in zusammenhang mit der persistenz und/oder anhäufung von prenylierten proteinen
Combination of an HMG-CoA reductase inhibitor and a farnesyl-pyrophosphatase synthase inhibitor for the treatment of diseases related to the persistence and/or accumulation of prenylated proteins

(30) Priorité: 05.07.2006 FR 0606097
(43) Date de publication de la demande: 23.09.2015
(62) Demande divisionnaire de: 07803849.4
(73) Titulaire: Université d'Aix-Marseille, 13284 Marseille Cedex 13 (FR); Association Francaise Contre Les Myopathies (AFM), 75651 Paris Cedex 13 (FR); Assistance Publique Hôpitaux de Marseille, 13354 Marseille Cedex 5 (FR); Universidad de Oviedo, 33003 Oviedo (ES)
(72) Inventeur: Levy, Nicolas, 13008 MARSEILLE (FR); Cau, Pierre, 13540 PUYRICARD (FR); Lopez-Otin, Carlos, 33005 OVIEDO (Asturias) (ES)
(74) Mandataire: Novagraaf Technologies

(56) Documents cités:
- EP-A1- 1 127 573
- FONG L G ET AL: "A protein farnesyltransferase inhibitor ameliorates disease in a mouse model of progeria", SCIENCE, AMERICAN ASSOCIATION FOR THE ADVANCEMENT OF SCIENCE, US, vol. 311, 17 mars 2006 (2006-03-17), pages 1621-1623, XP002420824, ISSN: 0036-8075, DOI: 10.1126/SCIENCE.1124875
- GORDON L B ET AL: "Reduced adiponectin and HDL cholesterol without elevated C-reactive protein: Clues to the biology of premature atherosclerosis in Hutchinson-Gilford Progeria Syndrome", JOURNAL OF PEDIATRICS, MOSBY-YEAR BOOK, ST. LOUIS, MO, US, vol. 146, no. 3, 1 mars 2005 (2005-03-01), pages 336-341, XP004817122, ISSN: 0022-3476, DOI: 10.1016/J.JPEDS.2004.10.064

## Description

La présente invention se place dans le domaine du traitement de situations pathologiques ou non, liées à l'accumulation et/ou la persistance dans les cellules de protéines prénylées.

Le noyau des cellules eucaryotes est délimité par une double membrane percée de pores, l'enveloppe nucléaire, qui contrôle les échanges moléculaires entre les deux compartiments nucléaire et cytoplasmique. Cette enveloppe isole partiellement le contenu du noyau, c'est-à-dire le matériel génétique et toute la machinerie enzymatique nécessaire aux fonctions du génome nucléaire.

L'enveloppe nucléaire est constituée de 2 membranes concentriques, la membrane externe, en continuité avec le réticulum endoplasmique, et la membrane interne. Cette dernière est bordée sur sa face interne par un maillage fibrillaire dense appelé *lamina* nucléaire. Il s'agit d'un réseau protéique essentiellement composé de polymères de lamines et de protéines associées. Chez les vertébrés, on distingue deux sous-classes de lamines : les lamines de type A (lamines A et C), et de type B (lamines B1, B2 et B3) qui participent toutes à l'élaboration de la *lamina.* Cette dernière est maintenue en place par l'association avec d'autres protéines, fixées à la membrane interne de l'enveloppe nucléaire (pour revue, Gruenbaum & al. 2005).

Les lamines sont des protéines en forme de filament appartenant à la famille des filaments intermédiaires (type V) qui ont toutes une structure commune : un court segment globulaire N-terminal (tête) séparé d'un autre segment globulaire C-terminal (queue) par un long domaine central organisé en plusieurs hélices *alpha (rod domain).* La queue globulaire contient notamment un signal de localisation nucléaire (*NLS*) permettant l'adressage au noyau après la synthèse. Le domaine central permet l'association de deux molécules de lamine parallèles et leur organisation en filaments par association des dimères "tête-bêche". Cette structure leur confère des propriétés mécaniques très résistantes.

Seules la lamine A et les lamines B subissent une maturation après la synthèse d'un précurseur (pour revue, Gruenbaum & al. 2000). La lamine C est directement synthétisée sous sa forme mature.

Le précurseur de la lamine A et des lamines B se termine par un motif *CaaX* caractéristique (C est une cystéine, a un acide aminé à chaîne aliphatique non chargée et *X* un acide aminé quelconque, ici une méthionine, pour revue, Lévy & Cau 2003).

Le motif *CaaX* C-terminal permet la fixation d'un acide gras (en général, un acide gras en C15, farnésyle) grâce à une farnésyl-transférase. Cette prénylation (le motif farnésyle dérive d'une unité aliphatique de base en C5 appelée isoprène) permet aux prélamines de venir s'insérer dans la membrane du réticulum endoplasmique après leur synthèse dans le cytosol. Elles y subissent l'action d'une endoprotéase elle-même insérée dans la membrane d'enveloppe du réticulum et dont le site actif est cytosolique. L'endoprotéase spécifique de la prélamine A est Face1 (ou ZMPSTE24 (*Zinc Metallo-Protease* homologue de STE24 de levure), tandis que Face2 (ou Rce1, *ras-converting enzyme*) est spécifique des prélamines B. Ces enzymes catalysent l'hydrolyse de la liaison peptidique entre la cystéine et l'aminoacide suivant (aliphatique), raccourcissant les prélamines de 3 acides aminés. L'extrémité carboxyle de la cystéine farnésylée est ensuite reconnue par une isoprénylcystéine-carboxyméthyl transférase (ICMT) qui vient y fixer un groupement méthyle par estérification.

Seule la maturation de la prélamine A se poursuit avec un deuxième clivage endoprotéolytique par Face1, qui libère un farnésyl-peptide de 15 acides aminés et la lamine A mature. Cette lamine A, qui ne comporte plus l'acide gras, devient soluble, est importée dans le noyau grâce à son signal de localisation nucléaire, et se localise dans la *lamina* nucléaire elle-même ainsi que dans le reste du compartiment nucléaire, constituant un véritable squelette nucléaire (Gruenbaum & al. 2005). La lamine B mature en revanche possède toujours à l'extrémité C-terminale sa cystéine farnésylée et méthylestérifiée. Elle reste donc insérée dans la membrane d'enveloppe du réticulum, puis dans la face nucléoplasmique de l'enveloppe nucléaire, d'où sa localisation exclusive à la *lamina* nucléaire, sous la membrane interne de l'enveloppe nucléaire où elle est ancrée.

Sous le terme de prénylation on entend la fixation au groupe thiol d'une cystéine soit d'une chaîne farnésyle de 15 atomes de carbone, on parle alors de farnésylation, soit d'une chaîne géranyl-géranyle de 20 atomes de carbone, on parle alors de géranyl-géranylation (Reid & al. 2004), ou de tout autre dérivé de l'isoprène.

La farnésylation, catalysée par la farnésyl-transférase (FTase) qui reconnaît la séquence consensus C-terminale (*CaaX)*, fixe préférentiellement un groupement farnésyle sur le résidu cystéine du motif.

La géranyl-géranylation est la fixation par la géranylgéranyl-transférase (GGTase) d'un groupement géranyl-géranyle sur le résidu cystéine du motif.

Ces acides gras sont issus de la voie de biosynthèse, qui, à partir de l'hydroxyméthyl-glutaryl-Coenzyme A, est utilisée par les cellules pour fabriquer notamment le cholestérol, les stéroïdes, l'hème de l'hémoglobine et les ubiquinones (Hampton & al. 1996).

La famille des protéines prénylées comporte environ 300 membres dans le génome humain, dont la majorité est identifiable par le motif C-terminal C*aaX* (Reid & al. 2004). Les protéines des familles *Ras, Rho, Rab* (Leung & al. 2006) certaines protéines assurant une fonction d'import vers la mitochondrie (HDJ2), certaines protéines mitotiques (CENPE, CENPF) sont notamment prénylées (Winter-Vann & Casey 2005). En général, si dans le motif *CaaX* X est une sérine, une méthionine, une cystéine, une alanine ou un glutamate, l'isoprénoïde préférentiellement greffé est le farnésyle. Si X est une leucine, la reconnaissance du motif *CaaL* se fera préférentiellement par la GGTase, qui catalysera le transfert d'un groupement géranyl-géranyle (Basso & al. 2006). Il est probable que d'autres groupements dérivés de l'isoprène peuvent aussi être fixés sur cette cystéine, bien que cela ne soit pas décrit dans la littérature.

Chez l'homme, il existe trois gènes de lamines. Le gène *LMNA,* situé en 1q21.2-q21.3 (Wydner & al. 1996), donne par épissage alternatif les lamines A et C. Le gène *LMNA* est composé de 12 exons. Le début de l'exon 1 code l'extrémité globulaire N-terminale commune aux lamines A et C ; la fin de l'exon 1 et jusqu'au début de l'exon 7 codent la partie hélicoïdale centrale ; enfin, les autres exons codent l'extrémité globulaire C-terminale (Lévy & Cau 2003).

En fait, le gène code pour 4 produits épissés différemment, dont les lamines C et la prélamine A sont les 2 principaux (Lin & Worman 1993). La production différentielle des lamines A et C se fait par l'utilisation d'un site d'épissage alternatif au niveau de l'exon 10 du pré-messager, de telle sorte que la lamine C est codée par les exons de 1 à 10 et la lamine A est codée par les exons de 1 à 9, les 90 premières paires de bases de l'exon 10, et les exons 11 et 12 (lamine A-spécifiques).

Par conséquent, les peptides prélamine A et lamine C sont identiques au niveau des premiers 566 acides aminés, les extrémités C-terminales des lamines C et de la prélamine A contenant ensuite respectivement 6 et 98 acides aminés spécifiques.

Les lamines de type B comprennent trois protéines différentes (Shelton & al. 1981) : les lamines B1, B2 (les deux isoformes les plus représentées) et B3. Le gène *LMNB1* est situé en 5q23.3-q31.1 et comporte 11 exons codant la lamine B1 (Lin & Worman 1995). Le gène *LMNB2* est localisé en 19p13.3 et code pour les lamines B2 et B3 par un mécanisme d'épissage alternatif (Biamonti & al. 1992).

Les lamines de type B sont exprimées constitutivement dans toutes les cellules à partir des premiers stades de développement, tandis que les lamines de type A sont en général absentes dans les cellules souches embryonnaires (Rober et al. 1989, Stewart et al. 1987) et sont exprimées dans toutes les cellules somatiques différenciées. Leur expression est sujette à des régulations selon le tissu et au cours de la vie (Duque & al 2006). Il semble que leur expression ne soit pas nécessaire, puisque des souris chez lesquelles on a bloqué spécifiquement l'expression de lamine A, mais qui expriment tout de même la lamine C et les autres lamines, n'ont pas de phénotype apparent (Fong & al 2006).

Les lamines interagissent avec un nombre très élevé de partenaires protéiques ayant des fonctions très diverses; elles sont par conséquent impliquées dans un grand nombre de processus nucléaires, incluant la réplication et la réparation de l'ADN, le contrôle de la transcription et de l'épissage, l'organisation de la structure chromatinienne (pour revue, voir Shumaker & al. 2003, Zastrow & al. 2004, Hutchison & al. 2004, Gruenbaum & al. 2005). Les altérations de la structure de la *lamina* sont à l'origine de nombreuses pathologies héréditaires humaines. Elles sont dues à des mutations des gènes codant les lamines, ou d'autres protéines de la *lamina.* On a regroupé ces pathologies sous le terme générique de laminopathies (Broers & al. 2006, Mattout & al 2006). Récemment, des mutations dans les gènes des enzymes responsables de la maturation des lamines (Face1 notamment), ont été identifiées, donnant lieu à des pathologies appartenant également au groupe des laminopathies (Navarro & al 2004 et 2005).

A ce jour, la seule pathologie chez l'homme associée à des mutations des gènes *LMNB1* ou 2 est une leucodystrophie causée par une duplication complète du gène LMNB1 (Padiath & al 2006). Un doute subsiste sur l'implication potentielle de variations de séquences trouvées dans LMNB2 chez des patients atteints du syndrome de Barraquer-Simon (Hegele & al 2006). Cependant, il a été démontré *in vitro* par des expériences de RNAi (RNA-interférence) (Harborth & al. 2001), ainsi que chez le modèle murin (Vergnes & al. 2004), que les lamines de type B sont essentielles pour le développement et l'intégrité cellulaire. En effet, la déficience en lamine B1 entraîne chez la souris une létalité périnatale. Par ailleurs, les noyaux des fibroblastes embryonnaires des mêmes souris *LMNB1* déficientes montrent des altérations remarquables de la morphologie nucléaire, proches de celles observées chez les patients porteurs de mutations du gène *LMNA.* De plus, il a été montré récemment que les lamines B sont nécessaires à la formation du fuseau de division pendant la mitose, ce qui tend à prouver que leur rôle est dynamique et multiple au cours du cycle cellulaire, et pas uniquement restreint au maintien de l'architecture du noyau (Tsai & al. 2006). Sur ce dernier rôle, un article récent constitue la démonstration de la fonction structurale des lamines B : des cellules artificiellement privées de lamines B1 ont un noyau "flottant" dans la cellule, qui tourne sur lui-même (Li & al 2007). La redondance fonctionnelle existant entre les deux lamines B1 et B2 est sans doute aussi le reflet direct de leur indispensabilité, exerçant une forte pression de sélection et masquant l'effet de mutations éventuelles de la séquence des gènes correspondants.

Les altérations fonctionnelles des lamines A/C, dues à des mutations du gène *LMNA,* sont à l'origine d'au moins 15 désordres regroupant des pathologies très diverses dans un spectre clinique allant de formes peu sévères, affectant un seul tissu de façon isolée, à des formes systémiques létales en période périnatale.

Nombre de mutations du gène *LMNA* modifient de façon notable l'assemblage des protéines dans l'enveloppe nucléaire et en perturbent le fonctionnement. Dans les cellules de divers tissus, la morphologie des noyaux est altérée : ils présentent souvent des hernies qui extrudent du matériel génétique dans le cytoplasme (Goldman & al. 2004).

Les protéines habituellement associées à l'enveloppe nucléaire, les lamines B, certaines protéines des pores nucléaires et les protéines LAP2, sont absentes du pourtour de ces hernies.

Ces anomalies morphologiques sont suivies d'altérations fonctionnelles, qui finissent par entraîner la mort cellulaire. Parmi l'ensemble des pathologies regroupées sous la dénomination de laminopathies, seules celles liées à l'accumulation anormale d'une forme prénylée de protéine sont concernées par la présente invention.

Ce sont principalement le syndrome de Hutchinson-Gilford, ou Progéria (De Sandre-Giovannoli & al. 2003, Eriksson & al. 2003), et la dermopathie restrictive (Navarro & al. 2004). Dans ces 2 syndromes, la cause physiopathologique est une accumulation et une persistance dans les cellules des malades de prélamine farnésylée non maturée.

La dermopathie restrictive, létale autour de la période natale, se caractérise par des signes cliniques qui sont presque tous la conséquence d'un déficit cutané qui restreint les mouvements *in utero.* Cette pathologie est très rare. La peau est rigide et tendue, elle cède par endroits, provoquant par exemple des déchirures au niveau des aisselles ou du cou. Les cils, les sourcils et le duvet cutané sont absents ou très clairsemés. Un hydramnios est souvent présent, et la diminution des mouvements foetaux est signalée dès le 6ème mois de grossesse. Au niveau du squelette, la radiographie révèle des contractures de toutes les articulations, des pieds en piolet, des clavicules fines, dysplasiques et bi-partites, des côtes en ruban, des os longs tubulaires des bras et une déminéralisation au niveau du crâne. La transmission de la dermopathie restrictive létale est récessive autosomique.

Des mutations de LMNA et de ZMPSTE24/Face1 ont été rapportées pour cette pathologie (Navarro & al. 2004). Dans les 2 cas, le mécanisme physiopathologique est le même : la prélamine A ne peut pas maturer (mutation nulle de Face1 ou disparition du site de clivage par mutation de la prélamine A), et reste farnésylée, et donc insérée dans la membrane nucléaire. L'accumulation et la persistance dans les cellules de ces précurseurs anormaux, qui empêchent probablement l'interaction normale des lamines B et C avec leurs partenaires, entraînent la mort des cellules et, à brève échéance, du patient. Il a été clairement démontré que c'est bien la persistance du groupement farnésyle, et non l'absence de lamine A mature, comme on pourrait le penser de prime abord, qui est responsable de la toxicité cellulaire (Fong & al. 2004).

En avril 2003, à partir d'un recoupement des symptômes communs à la dysplasie acromandibulaire et à certaines maladies se traduisant par un vieillissement prématuré, les inventeurs ont montré que la Progéria, la forme la plus typique et la plus grave de vieillissement précoce, résulte d'une mutation du gène *LMNA* (De Sandre-Giovannoli & al. 2003). Les enfants atteints par cette maladie, aussi nommée syndrome de Hutchinson-Gilford, souffrent d'un vieillissement accéléré, jusqu'à dix fois plus rapide que celui d'un individu normal, et ont une espérance de vie qui ne dépasse pas 13 ans. En France, un enfant sur environ six millions est touché. Les symptômes sont un vieillissement cutané, une calvitie, une réduction de la taille de la mâchoire et des problèmes liés à la vieillesse, par exemple, une raideur des articulations et des troubles cardio-vasculaires. Ces derniers, tels l'infarctus du myocarde ou l'athérosclérose, sont souvent la cause de la mort.

Le document EP 1 127 573 29 décrit des compositions et méthodes de traitement de l'ostéoporose. En particulier, il décrit une composition comprenant conjointement une statine et un polyphosphonate pour diminuer le taux de cholestérol chez des lapins. Cette composition est décrite comme susceptible d'être utilisé dans le traitement d'une perte osseuse (par exemple ostéoporose) et/ou le traitement de l'athérosclérose.

La mutation incriminée, située dans l'exon 11 du gène *LMNA,* active un site cryptique d'épissage du pré-ARNm, conduisant à un ARNm délété de 150 nucléotides (De Sandre-Giovannoli & al. 2003, Eriksson & al. 2003). Cet ARNm délété est traduit en une prélamine A anormale, la progérine, qui ne peut pas être maturée en lamine A normale : l'absence de 50 acides aminés de l'exon 11 comportant le site de reconnaissance de la protéase bloque le 2^{e} clivage de la progérine dont l'extrémité C-terminale conserve son groupement farnésyle. Elle reste donc insérée dans la face nucléoplasmique de l'enveloppe nucléaire, qui présente les altérations caractéristiques, hernies du nucléoplasme dans le cytosol et anomalies de la répartition de l'hétérochromatine périphérique (Goldman & al. 2004). Ici encore, c'est la persistance du groupe farnésyle, par ailleurs nécessaire pour l'ancrage à la membrane d'enveloppe du réticulum dans laquelle sont localisées certaines des enzymes responsables de la maturation (clivages, méthylation) qui est responsable de la toxicité cellulaire de la progérine (Fong & al. 2004).

Ces pathologies systémiques présentent la particularité d'être associées à l'apparition précoce de signes habituellement liés au vieillissement. Leur caractéristique physiopathologique commune est de générer une lamine prénylée, avec les conséquences décrites.

Deux études récentes ont montré qu'une réduction de l'accumulation intranucléaire de la prélamine farnésylée, tronquée ou non, prévient efficacement l'apparition du phénotype cellulaire. La première a été réalisée sur le modèle murin progéroïde déficient en protéase Face1 (Pendas & al. 2002). Lorsqu'elles sont croisées avec des souris exprimant moitié moins de Lamine A (souris *Lmna* +/-), les effets de l'absence de Face1 sont amoindris (Varela & al. 2005). La deuxième étude montre que le traitement de cellules de patients HGPS par des *morpholino* (oligonucléotides antisens) ciblant le site d'épissage cryptique abolit le phénotype mutant (Scaffidi & Misteli 2005).

Plusieurs études récentes (Scaffidi & Mistelli 2006, Cao & al 2007) montrent l'implication de la lamine A dans le processus de vieillissement physiologique. En particulier, il a été démontré qu'au cours du vieillissement physiologique, une lamine A aberrante s'accumule au cours du temps à la périphérie du noyau des cellules. Cette lamine aberrante est en fait de la progérine, la cellule au cours de sa vie et de son fonctionnement normal utilisant accidentellement de temps en temps le site d'épissage cryptique de l'exon 11, la progérine produite s'accumule petit à petit sous la *lamina.* Finalement, la cellule âgée «normale» peut présenter des hernies caractéristiques d'une laminopathie causée par ces événements d'épissage accidentel, qui entraînent sa mort.

Il apparaît que des mécanismes moléculaires identiques sont d'une part responsables des signes de vieillissement prématuré des individus atteint de Progéria et d'autre part, à un niveau beaucoup plus faible, interviennent dans le vieillissement physiologique d'individus non porteurs de mutations.

Il existe dans l'art antérieur deux approches thérapeutiques décrites pour améliorer le phénotype cellulaire causé par la production pathologique de progérine. La première de ces solutions est tout simplement d'empêcher l'utilisation par le spliceosome de ce site d'épissage cryptique dans l'exon 11, en le "masquant" par traitement avec un oligonucléotide antisens (Scaffidi & Misteli 2005), ou avec un rétrovirus produisant un siARN (Huang & al 2005). Les résultats sont prometteurs *in vitro,* mais il s'agit ici de thérapie "génique", et le développement d'un médicament autour de cette approche est immanquablement long et compliqué, avec tous les inconvénients liés à la vectorisation des OAS pour obtenir un effet *in vivo.* La deuxième solution consiste à inhiber la farnésyl-transférase, l'enzyme qui catalyse le transfert du groupement farnésyle sur les prélamines à partir de farnésyle-pyrophosphate. Lorsque de tels inhibiteurs (FTI) sont utilisés, une enveloppe nucléaire «normale» n'est que partiellement restaurée sur des cellules HGPS (Progéria) en culture, et la survie de souris RD (KO ZMPSTE24) est améliorée (Glynn & Glover 2005, Capell & al. 2005, Toth & al. 2005, Fong & al. 2006).

Cependant, le blocage de la farnésylation peut induire une géranyl-géranylation compensatoire (Bishop & al. 2003).

D'autre part, il a été rapporté récemment que les FTI induisent un arrêt du cycle cellulaire en bloquant le protéasome (Demyanets & al. 2006, Efuet & Keyomarsi 2006). Ainsi, le traitement induit sans doute une accumulation dans le nucléoplasme de progérine probablement ubiquitinylée non dégradée par le protéasome.

Par ailleurs, des travaux récents rapportent que la diminution du taux de farnésylation de la progérine *in vivo* est très faible, de l'ordre de 5 % (Young & al 2006), ce qui ne suffit pas à expliquer la restauration de la morphologie nucléaire observée *in vitro.*

Finalement, les FTI sont spécifiques d'une seule des voies de prénylation protéique, et ne peuvent être envisagés comme inhibiteurs globaux des prénylations post-traductionnelles.

Par ailleurs, il est rapporté que l'absence totale d'une des enzymes de cette voie, la mévalonate-kinase, est létale durant l'enfance (mutation homozygote perte de fonction du gène codant pour cette enzyme, syndrome rapporté par Hoffmann & al 2003).

Après de longues recherches, les inventeurs ont montré que l'association d'un inhibiteur de l'hydroxyméthylglutaryl-coenzyme A réductase (famille des statines) et d'un inhibiteur de la farnésyl-pyrophosphate synthase (famille des amino-biphosphonates, NBP), ou de l'un de leurs sels physiologiquement acceptable, est un traitement efficace des situations, pathologiques ou non, liées à l'accumulation et/ou la persistance dans les cellules de protéines prénylées, en ce sens qu'elle bloque l'ensemble de la voie de prénylation des protéines, tant en C15 qu'en C20 ou dans les formes non caractérisées. Les inventeurs ont en outre constaté que l'association d'un inhibiteur de l'hydroxyméthylglutaryl-coenzyme A (HMG-CoA) réductase et d'un inhibiteur de la farnésyl-pyrophosphate synthase présente un effet synergique dans la restauration du phénotype normal chez des fibroblastes de patients atteints de Progéria. L'effet de l'association est nettement supérieur à ce qu'est l'effet de l'un ou l'autre des inhibiteurs utilisé individuellement.

L'utilisation de l'association sur des cellules de patients atteints de Progéria conduit à une inhibition de la prénylation des protéines, donc à l'apparition de prélamine A non farnésylée et à l'amélioration des symptômes nucléaires.

Ces résultats permettent d'envisager l'utilisation d'un inhibiteur de l'hydroxyméthylglutaryl-coenzyme A (HMG-CoA) réductase et d'un inhibiteur de la farnésyl-pyrophosphate synthase, ou de l'un de leurs sels physiologiquement acceptable, dans la préparation d'une composition, particulièrement d'une composition pharmaceutique, destinée au traitement des situations, pathologiques ou non, liées à l'accumulation et/ou la persistance dans les cellules de protéines prénylées.

L'invention a ainsi pour objet une composition comprenant un inhibiteur de l'hydroxyméthylglutaryl-coenzyme A (HMG-CoA) réductase et d'un inhibiteur de la farnésyl-pyrophosphate synthase, ou de l'un de leurs sels physiologiquement acceptable, pour son utilisation dans le traitement des situations, pathologiques impliquant des signes de vieillissement, qu'il soit naturel, prématuré ou accéléré choisies parmi la détérioration de l'endothélium vasculaire le vieillissement de la peau, et la lyse osseuse, liées à l'accumulation et/ou la persistance dans les cellules de progérine et/ou de prélamine A farnésylée.

Il est aussi dans la portée de l'invention d'utiliser des composés qui sont à la fois des inhibiteurs de l'hydroxyméthylglutaryl-coenzyme A (HMG-CoA) réductase et des inhibiteurs de la farnésyl-pyrophosphate synthase. L'invention a également pour objet l'utilisation non thérapeutique de la composition comprenant un inhibiteur de l'hydroxyméthylglutaryl-coenzyme A (HMG-CoA) réductase et d'un inhibiteur de la farnésyl-pyrophosphate synthase, ou de l'un de leurs sels physiologiquement acceptable, dans le traitement de situations non pathologiques impliquant des signes de vieillissement naturel choisies parmi la détérioration de l'endothélium vasculaire et le vieillissement de la peau, liées à l'accumulation et/ou la persistance dans les cellules de progérine et/ou de prélamine A farnésylée.

Dans la présente, il est également décrit que la composition selon l'invention peut être destinée au traitement des situations, pathologiques ou non, liées à l'accumulation et/ou la persistance dans les cellules de progérine, encore plus particulièrement au traitement des situations liées à l'accumulation et/ou la persistance dans les cellules de prélamine A farnésylée, que celle-ci soit ou non tronquée ou modifiée.

Notamment, puisqu'il est admis que le vieillissement physiologique fait intervenir l'accumulation de progérine dans les cellules au cours de la vie et que la progérine se concentre en particulier dans les cellules mésenchymateuses, la composition selon l'invention peut être destinée à prévenir les effets du vieillissement cellulaire, au niveau de la peau et/ou de l'endothélium vasculaire.

La composition selon l'invention pourra être destinée au traitement de tout être vivant, homme ou animal, particulièrement pour la prévention des effets du vieillissement cellulaire. La composition trouve donc une application aussi bien en médecine humaine qu'en médecine vétérinaire.

Selon l'invention, tout inhibiteur de la farnésyl-pyrophosphate synthase, ou l'un de ses sels physiologiquement acceptable, peut être utilisé dans la préparation de la composition selon l'invention.

Les sels physiologiquement acceptables peuvent être par exemple des sels formés avec les acides chlorhydrique, bromhydrique, nitrique, sulfurique, phosphorique, les acides carboxyliques tels que par exemple les acides acétique, formique, propionique, benzoïque, maléique, fumarique, succinique, tartrique, citrique, oxalique, glyoxylique, aspartique, alcane sulfonique tels que les acides méthane ou éthane sulfoniques, arylsulfonique tels que les acides benzène ou paratoluène sulfoniques.

Particulièrement l'inhibiteur de la farnésyl-pyrophosphate synthase peut être un des membres de la famille des polyphosphonates, particulièrement des aminobiphosphonates (NBP), ou l'un de ses sels physiologiquement acceptable.

Les polyphosphonates sont des molécules synthétiques très utilisées dans le traitement de l'ostéoporose et de la régénération osseuse.

Le terme de phosphonate s'applique à des molécules très semblables au phosphate :

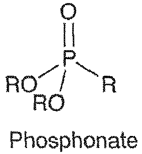

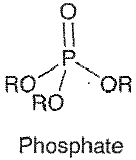

Le coeur des biphosphonates (BP) est l'équivalent d'une liaison P-O-P comme dans l'ATP par exemple, mais où l'oxygène est remplacé par un carbone. Ceci confère une stabilité tout à fait particulière à ces molécules.

Un biphosphonate simple serait équivalent à l'ADP, les 2 groupes phosphates (O₃P-) étant remplacés par le groupe biphosphonate

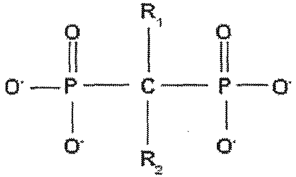

Le carbone central, à la différence de l'oxygène des phosphates, peut encore être impliqué dans 2 liaisons, et c'est la nature des groupements greffés sur ce carbone qui fait la spécificité des biphosphonates.

Lorsque les chaînes "latérales" (R1 et R2) comportent une fonction amine (NH), ou plus généralement un ou plusieurs atomes d'azote, on parle d'aminobiphosphonate, ou NBP.

Bien sûr, d'autres substituants peuvent être fixés sur les oxygènes.

L'acide pyrophosphorique, ou pyrophosphate en solution (PPi) 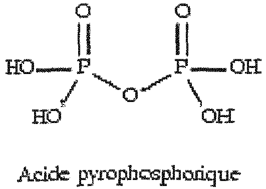 est utilisé dans de nombreuses réactions métaboliques comme transporteur de substrats, et il est restitué à la fin de la réaction. L'une des voies métaboliques utilisant des molécules couplées au pyrophosphate est celle de la prénylation protéique.

Le greffage d'un isopentènyl-PP (unité de base en C5) sur un géranyl-PP (C10) pour donner du farnésyl-PP, réaction catalysée par l'enzyme farnésyl-pyrophosphate synthase (FPS), libère un PPi.

C'est cette étape qui est spécifiquement inhibée par les NBP.

A cet égard et à titre d'exemple, l'aminobiphosphonate (inhibiteur de la farnésyl-pyrophosphate synthase) peut être choisi parmi
- l'acide alendronique ou sa forme ionique, l'alendronate ;
- l'acide ibandronique, ou sa forme ionique, l'ibandronate ;
- l'acide néridronique ou sa forme ionique, le néridronate ;
- l'acide olpadronique ou sa forme ionique, l'olpadronate ;
- l'acide pamidronique ou sa forme ionique, le pamidronate ;
- l'acide risédronique ou sa forme ionique, le risédronate ;
- l'acide zolédronique ou sa forme ionique le zolédronate ;
- l'acide 4-N,N-diméthylaminométhane diphosphonique ou sa forme ionique le diméthylaminométhanediphosphonate ;
- l'α-amino-(4-hydroxybenzylidène) diphosphonate.

Préférentiellement selon l'invention, on préfère utiliser l'acide zolédronique (aussi appelé acide zolendronique) ou sa forme ionique, le zolédronate (aussi appelé zolendronate).

Selon l'invention, tout inhibiteur de HMG-CoA réductase, ou l'un de ses sels physiologiquement acceptable, peut être utilisé dans la préparation de la composition.

Particulièrement l'inhibiteur de HMG-CoA réductase peut être une molécule de la famille des statines, qu'elle soit liposoluble ou hydrosoluble, ou l'un de ses sels physiologiquement acceptable.

Les statines ont été mises en évidence chez des champignons. Elles ont une activité inhibitrice de l'HMG-CoA réductase, enzyme clé de la biosynthèse du cholestérol et des stéroïdes, qui catalyse la réduction de l'hydroxyméthyl-glutarate couplé au Coenzyme A en acide mévalonique (mévalonate en solution). Cette inhibition est assurée par leur analogie structurale avec le squelette de l'hydroxyméthylglutarate. La voie métabolique impliquée est certes celle de la biosynthèse du cholestérol, mais c'est aussi celle de la synthèse des groupes prényles, des polymères de l'unité de base à 5 carbones isoprène utilisés pour modifier environ 300 protéines dans les cellules et leur attacher une queue lipophile, permettant notamment leur ancrage dans les membranes.

Les principaux polyprènes, tous issus du pyruvate et de l'HMG-CoA, sont le géranyle (C10), le farnésyle (C15) et le géranyl-géranyle (C20).

Toutes les statines sont globalement hépatosélectives, mais toutes n'ont pas le même mode d'entrée dans les cellules. En effet, pravastatine et rosuvastatine sont toutes 2 hydrophiles, donc hydrosolubles, au contraire de toutes les autres qui sont lipophiles, donc peuvent diffuser librement à travers les membranes plasmiques (bicouches lipidiques), ce qui explique sans doute leur plus haute toxicité. Les statines hydrosolubles ont besoin d'un transporteur spécifique pour entrer dans la cellule, *Organic Anion Transporter 3,* ou OAT3, ouSLC22A8 (Takedaa & al 2004).

Elles sont très utilisées pour le traitement de l'hypercholestérolémie, et leurs effets secondaires, rares, sont bien caractérisés. Ce sont notamment des cas de rhabdomyolyse (1 à 7% des cas selon la molécule utilisée, Evans & al 2002), dont le signe avant-coureur, des douleurs musculaires chez le patient traité, entraîne l'arrêt immédiat du traitement.

A cet égard et à titre d'exemple, une statine peut être choisie parmi l'atorvastatine, la simvastatine, la pravastatine, la rivastatine, la mévastatine (ou compactine), la fluindostatine, la vélostatine, la fluvastatine, la dalvastatine, la cérivastatine, la pentostatine, la rosuvastatine, la lovastatine, la pitavastatine, ou de l'un de leurs sels physiologiquement acceptable.

La lovastatine, la pravastatine et la simvastatine sont les molécules dérivées de métabolites fongiques, tandis que les autres, (atorvastatine, cérivastatine, fluvastatine, pitavastatine et rosuvastatine) sont entièrement synthétiques. Préférentiellement selon l'invention on utilise la pravastatine, une statine semi-naturelle hydrosoluble.

Bien entendu il est possible selon l'invention d'utiliser un, voire deux ou plusieurs inhibiteur(s) de la farnésyl-pyrophosphate synthase associés à un, voire deux ou plusieurs inhibiteur(s) de HMG-CoA réductase.

Dans la présente, il est également décrit que la composition peut être destinée au traitement de situations, pathologiques ou non, nécessitant d'inhiber la prénylation des protéines. Ces pathologies peuvent être étiquetées ou non, par exemple le syndrome de Costello, de Noonan, le syndrome cardio-fascio-cutané, ou les maladies liées à une prénylation anormale ou persistante de Ras et des protéines de transduction du signal.

Selon l'invention, la composition peut être destinée au traitement de situations, pathologiques, présentant des signes de vieillissement, qu'il soit naturel, prématuré ou accéléré choisies parmi la détérioration de l'endothélium vasculaire (protection de l'endothélium vasculaire). de vieillissement de la peau, et de lyse osseuse liées à l'accumulation et/ou la persistance dans les cellules de progérine et/ou de prélamine A farnésylée.

Dans la présente, il est également décrit que la composition selon l'invention est une composition pharmaceutique destinée au traitement de la Progéria (HGPS, Hutchinson-Gilford Progeria Syndrome) et de la dermopathie restrictive (DR ou RD).

Selon l'invention, l'inhibiteur de la farnésyl-pyrophosphate synthase et l'inhibiteur de la HMG-CoA réductase sont avantageusement présents dans la composition à des doses physiologiquement efficaces.

De manière générale, les doses à administrer peuvent être adaptées par l'homme du métier en fonction du patient, de la pathologie, du mode d'administration, etc. Il est entendu que des administrations répétées peuvent être réalisées, éventuellement en combinaison avec d'autres ingrédients actifs ou tout véhicule acceptable sur le plan physiologique (tampons, solutions saline, isotonique, en présence d'agents stabilisants, etc.).

En général la dose journalière des inhibiteurs sera la dose minimum pour obtenir l'effet thérapeutique souhaité.

Les compositions utilisant un inhibiteur de l'hydroxyméthylglutaryl-coenzyme A (HMG-CoA) réductase et un inhibiteur de la farnésyl-pyrophosphate synthase, selon l'invention, peuvent être formulées pour la voie digestive ou parentérale.

Lesdites compositions peuvent comprendre en outre au moins un autre ingrédient actif, particulièrement un autre ingrédient thérapeutiquement actif, pour une utilisation simultanée, séparée ou étalée dans le temps.

Selon l'invention, l'inhibiteur de l'hydroxyméthylglutaryl-coenzyme A (HMG-CoA) réductase et l'inhibiteur de la farnésyl-pyrophosphate synthase, peuvent être utilisés dans la composition, en mélange avec un ou plusieurs excipients ou véhicules inertes, c'est à dire physiologiquement inactifs et non toxiques. On peut citer par exemple des solutions salines, physiologiques, isotoniques, tamponnées, etc..., compatibles avec un usage physiologique et connues de l'homme du métier.

Les compositions peuvent contenir un ou plusieurs agents ou véhicules choisis parmi les dispersants, solubilisants, stabilisants, conservateurs, etc... Des agents ou véhicules utilisables dans des formulations (liquides et/ou injectables et/ou solides) sont notamment la méthylcellulose, l'hydroxyméthylcellulose, la carboxyméthylcellulose, les cyclodextrines, le polysorbate 80, le mannitol, la gélatine, le lactose, des huiles végétales ou animales, l'acacia, etc...

Les compositions peuvent être formulées sous forme de suspension injectable, de gels, huiles, comprimés, suppositoires, poudres, gélules, capsules, etc..., éventuellement au moyen de formes galéniques ou de dispositifs assurant une libération prolongée et/ou retardée. Pour ce type de formulation, on utilise avantageusement un agent tel que la cellulose, des carbonates ou des amidons.

L'administration peut être réalisée par toute méthode connue de l'homme du métier, de préférence par voie orale ou par injection, typiquement par voie intra-musculaire, intra-veineuse ou intra-péritonéale.

L'administration par voie orale est préférée.

S'agissant d'un traitement à long terme, la voie d'administration préférée sera sublinguale, orale ou transcutanée.
La Figure 1 illustre les résultats obtenus en Western Blot sur des fibroblastes témoins "normaux" traités avec des doses croissantes d'une statine hydrosoluble (pravastatine P, 20 à 100 µM), et d'un aminobiphosphonate (NBP zolédronate Z, 20 à 100 µM) ((Pistes A à I, respectivement P20/Z20, P20/Z60, P20/Z100, P60/Z20, P60/Z60, P60/Z100, P100/Z20, P100/Z60, P100/Z100)). La piste J est un contrôle positif de présence de prélamine A (fibroblastes de patients DR), la piste K est le contrôle négatif, traité avec le solvant seul (PBS).
La Figure 2 illustre les résultats obtenus aux doses efficaces de chacun des produits.
La Figure 3 illustre l'effet supérieur obtenu lors de l'administration des 2 produits ensemble.
La Figure 4 illustre l'action de l'association des 2 produits sur des cellules âgées.
La Figure 5 illustre l'action de l'association des 2 produits sur des souris modèle de Progéria (ne synthétisant pas l'enzyme Face1).
   5a: photographie représentative de souris âgées de 3 mois et respectivement sauvage, Zmpste24^{-/-} et Zmpste24^{-/-} traitée.
   5b: poids des souris sauvages (n=6), Zmpste24^{-/-} non traitées (n=7) et traitées (n=8), à 3 mois.
   5c: courbe de survie (type Kaplan-Meier) montrant l'allongement significatif de la survie de femelles traitées (n=7) (-◊-) par comparaison avec des souris non traitées (n=7) (-◆-).
   5d: image reconstituée en 3D de tomographie par scanner µCT de tibias de souris traitées et non traitées (en haut). En bas, représentation du volume osseux (à gauche, a) et du nombre de trabécules par millimètre carré (à droite, b).
   5e: quantification des anomalies nucléaires (%) sur des cellules de foie. A gauche, marquage fluorescent des noyaux au DAPI, à droite, comptage du nombre de noyaux anormaux.

Les exemples suivants servent à illustrer l'invention sans la limiter aucunement.

EXEMPLE 1 : Effet synergique de l'association d'un inhibiteur de l'hydroxyméthylglutaryl-coenzyme A (HMG-CoA) réductase hydrosoluble et d'un inhibiteur de la farnésyl-pyrophosphate synthase sur des cultures de cellules normales et progéroïdes

### A) PROTOCOLES

### ❖ Cellules et culture cellulaire

Les lignées de cellules sont soit des fibroblastes témoins AG16409 provenant du Coriell Institute, soit des fibroblastes issus de biopsies de patients atteints de Dermopathie Restrictive. Elles sont cultivées à 37°C sous 5% de CO₂ en salle P2.

Le milieu de culture complet usuel est
- RPMI (Invitrogen) complémenté avec
- Sérum de Veau Foetal 20% (Invitrogen)
- L-Glutamine 200 mM (Invitrogen)
- Mélange Pénicilline/Streptomycine/Fungizone 1 X (Stock 100 X, Cambrex)

### ❖ Récolte des cellules

La récolte des cellules est réalisée par trypsinisation de la façon suivante (protocole pour une grande flasque, 75 cm², BD Falcon) :
Le milieu est aspiré ;
Les cellules sont lavées avec 10 ml de PBS 1X (Invitrogen), 10 ml ;
5 ml d'une solution de Trypsine/EDTA 1X (Cambrex) sont ajoutés
La flasque est incubée environ 6 min à 37°C, le temps que les cellules se décollent ;
La trypsine est inhibée par dilution dans 15 ml de milieu complet ;
Les cellules sont culottées par centrifugation 10 min à 1000 tours par minutes à 16°C ;
Le culot est resuspendu dans 2 ml de PBS 1X, et re-centrifugé dans les mêmes conditions ;
Soit les cellules sont congelées pour une utilisation ultérieure, soit elles sont repiquées à partir de ce culot lavé.

### ❖ Traitements

La solution de pravastatine (statine hydrosoluble) utilisée est préparée comme suit :
40 mg de pravastatine (Sigma Aldrich, P4498) sont repris dans de l'eau stérile pour constituer une solution stock à 10 mM.

Les concentrations finales testées ont été de 500 nM, 1, 5, 50 et 100 µM, obtenues en diluant la solution stock dans du milieu complet.

La solution de zolédronate (NBP) utilisée est préparée comme suit :
Une solution stock d'acide (1-hydroxy-2-imidazol-1-yl-1-phosphono-éthyl) phosphonique (0,8 mg/ml, Novartis) est ajustée à une concentration de 2 mM.

Les concentrations finales testées ont été de 500 nM, 1, 5, 50 et 100 µM, obtenues en diluant la solution stock dans du milieu complet.

### ❖ Western blot

### ∘ Préparation des cellules

Pour une expérience de Western blot, les cellules sont traitées comme suit :
Environ 7,5 x 10⁵ cellules sont ensemencées dans une grande flasque et cultivées dans les conditions ci-dessus jusqu'à presque confluence (4 jours).

Au bout de 4 jours, les cellules sont lavées avec du PBS 1X, et reprise dans du milieu complet supplémenté avec le traitement.

Les cellules sont incubées le temps du traitement (de 6 à 72h, séquentiellement ou simultanément) dans l'incubateur à 37°C.

A l'issue du traitement, les cellules sont trypsinisées (protocole ci-dessus) et le culot obtenu stocké à -80 °C jusqu'à extraction des protéines.

### ∘ Extraction des protéines pour Western blot

Le culot cellulaire est repris dans 300 µl de tampon de lyse

| | | |
|---|---|---|
| Triton X100 | 1 | % |
| SDS | 0,1 | % |
| Désoxycholate de sodium | 0,5 | % |
| NaCl | 50 | mM |
| EDTA | 1 | mM |
| TrisHCl pH 7,4 | 20 | mM |
| Inhibiteur de Protéase (Roche 11697498001) | 1 | pastille pour 50 ml |

Extemporanément, on ajoute

| | | |
|---|---|---|
| Orthovanadate de sodium | 1 | mM |
| PMSF | 1 | mM |

Les cellules sont exposées à une sonication 2 fois 30 sec (*Brandson Sonifier Cell Disruptor B15*)*.*

Les débris cellulaires sont centrifugés pendant 10 min à 10 000 tours par minutes à 4 °C.

Le surnageant protéique est conservé à -80 °C jusqu'à utilisation.

Le dosage des protéines est effectué à la décongélation.

### ∘ Western blot

### ▪ Gel

Un gel d'acrylamide 8 % est classiquement utilisé pour détecter les différentes formes de lamines A/C.

| | | |
|---|---|---|
| Acrylamide/bisacrylamide 37/1 | 8 | % |
| TrisHCl pH 8,8 | 375 | mM |
| SDS | 0,1 | % |
| APS | 0,1 | % |
| TEMED | 0,01 | % |

Un gel de concentration est coulé sur le gel séparatif

| | | |
|---|---|---|
| Acrylamide/bisacrylamide 37,5/1 | 3 | % |
| TrisHCl pH 6.8 | 375 | mM |
| SDS | 0,1 | % |
| APS | 0,1 | % |
| TEMED | 0,01 | % |

La concentration en protéines des échantillons est dosée, et des aliquots sont ajustés à 50 µg par tube dans du tampon de lyse en qsp 15 µl.

5 µl de tampon de charge sont ajoutés à chaque échantillon

| | | |
|---|---|---|
| SDS | 4 | % |
| TrisHCl pH 6,8 | 100 | mM |
| Glycérol | 20 | % |
| β-mercaptoéthanol | 20 | % |
| Bleu de bromophénol | traces | |

Les échantillons sont dénaturés par chauffage 5 min à 95 °C et déposés dans les puits.

La migration a lieu à 50, puis 100 Volts, dans un tampon

| | | |
|---|---|---|
| Tris-Base | 0,3 | % |
| Glycine | 1,44 | % |
| SDS | 0,1 | % |

### ▪ Transfert

La membrane de transfert (Hybon P, Amersham Biosciences) est préparée par trempage dans l'éthanol, suivi d'un bain de 5 min dans l'eau stérile, et de 10 min dans le tampon de transfert :

| | | |
|---|---|---|
| Tris-Base | 12 | mM |
| Glycine | 96 | mM |
| Ethanol | 20 | % |

Le gel est humidifié pendant 20 min dans le tampon de transfert, puis le sandwich est monté (système Miniprotéan, Biorad).

Le transfert a lieu en général sur la nuit, en chambre froide, à 10 Volts.

La membrane est rincée dans du PBS 1X, conservée à l'humidité, et utilisé telle quelle pour la détection.

### ▪ Détection

La membrane est incubée 1h à température ambiante dans une solution de saturation :

| | | |
|---|---|---|
| Caséine | 10 | % |
| Tween 20 | 0,1 | % |
| PBS | 1 | X |

On la rince 2x 10 min dans du tampon de lavage (Tween 20 0,1% / PBS 1X).

L'anticorps primaire est dilué dans le tampon de saturation (détails et dilution, voir ci-dessous immunomarquage).

La membrane est incubée avec les anticorps primaires 1h à température ambiante sous agitation.

A l'issue, elle est rincée 3x avec du tampon de lavage, puis lavée 3x 15 min avec le même tampon.

L'anticorps secondaire (système couplé à la péroxydase, Jackson Immunoresearch) est dilué au 1/10000^{e} dans du tampon de saturation.

La membrane est incubée avec cette solution 30 à 45 min à température ambiante sous agitation.

A l'issue, elle est rincée 3x avec du tampon de lavage, puis lavée 3x 15 min avec le même tampon.

La détection est effectuée avec le kit ECL Plus Western Blotting System d'Amersham Bioscience, selon les indications du fournisseur.

Après révélation, la membrane est exposée sur film Biomax MR (Kodak), le temps nécessaire pour avoir un signal satisfaisant.

### ❖ Immunomarquage

### ∘ Préparation des cellules

Une culture de cellules est trypsinisée, et les cellules comptées sur cellule de Neubauer.

Des puits de culture style Labtech (Nunc, réf. 177399) sont ensemencés, à raison de 5x10⁴ cellules par puits.

Le milieu de culture complet est supplémenté par le ou les traitements (statine, NBP, les 2), et les cellules cultivées pendant le temps *ad hoc.*

A l'issue, le milieu de culture est aspiré, les puits démontés.

Les lames sont lavées dans du PBS 1X.

Les cellules sont fixées dans une solution de paraformaldéhyde 4% (dans PBS) pendant 10 minutes à température ambiante.

Un lavage de 10 min dans du PBS 1X est effectué.

Les cellules sont déshydratées par des bains successifs de 3 min dans des solutions de pourcentage éthanolique croissant (70, 90, 100%, ce dernier bain étant répété).

Après séchage, les lames sont stockées à -80°C jusqu'à utilisation.

### ∘ Marquage

Après décongélation, les cellules sont incubées 5 min à température ambiante en chambre humide dans 50 µl d'une solution de perméabilisation :

| | | |
|---|---|---|
| PBS | 1 | X |
| Triton X100 | 0,5 | % |
| RNS (Rabbit Normal Serum, Vector S5000) | 5 | % |
| Inhibiteur de Protéase (Roche 11697498001) | 1 | pastille pour 50 ml |

3 bains de préincubation de 15 min chacun sont effectués dans 50 µl de la solution d'incubation :

| | | |
|---|---|---|
| PBS | 1 | X |
| RNS | 5 | % |
| Inhibiteur de Protéase (Roche 11697498001) | 1 | pastille pour 50 ml |

L'anticorps primaire est dilué au 1/100^{e} dans 50 µl de solution d'incubation et mis au contact des cellules pendant 1h à température ambiante en chambre humide.

Les anticorps primaires utilisés sont de 2 types :
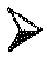 Souris anti-lamine A/C (côté N-terminal), clone 4A7, don de G. Morris (Oswestry, UK)
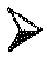 Chèvre anti-prélamine A (15 aa extrémité C-terminale), produit SC6214, Santa Cruz Biotechnology, Inc.

3 rinçages dans 50 µl de PBS 1X sont effectués pendant 15 min chacun.

L'incubation avec l'anticorps secondaire a lieu pendant 1h dans 50 ml de solution d'incubation à température ambiante en chambre humide. Les anticorps secondaires sont de deux types :
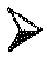 Ane anti-souris, Jackson Immunoresearch, dilution au 1/100^{e}
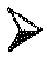 Ane anti-chèvre, Jackson Immunoresearch, dilution au 1/200^{e}

3 rinçages dans 50µl de PBS 1X sont effectués pendant 10 min chacun.

Une incubation avec 100 µl de solution DAPI 50 ng/ml (SERVA, réf. 18860) est réalisée pendant 15 min à température ambiante en chambre humide.

3 rinçages dans du PBS 1X sont effectués dans des bacs porte-lames pendant 5 min chacun.

Un ultime rinçage est effectué pendant 5 min dans une solution de Tween20 à 0,1% dans du PBS.

### ∘ Montage

Les cellules sont immergées dans une goutte de VectaShield (Vector), recouvertes d'une lamelle couvre-objet et observées sur un microscope à fluorescence (Leica DMR, Leica Microsystems), équipé d'un système de caméra coolSNAP (Princeton).

### B) RESULTATS

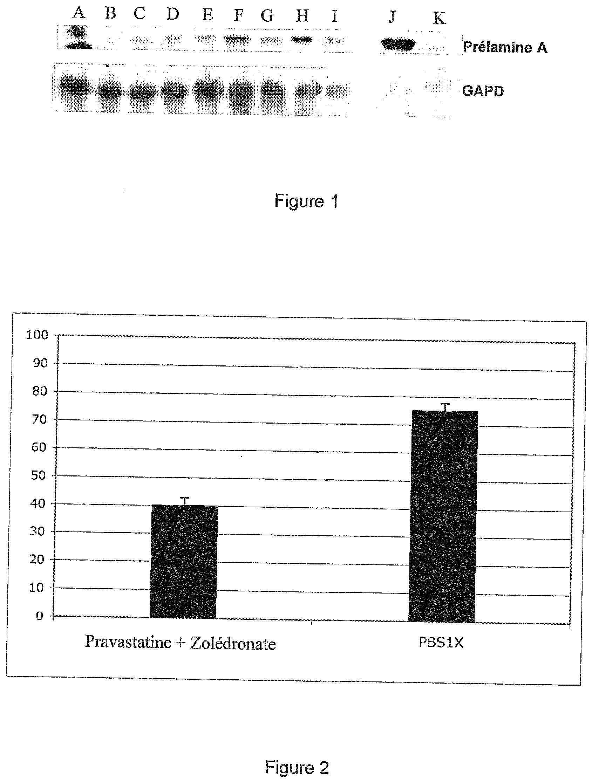

### B.1) Western blot (Figure 1)

Des fibroblastes témoins "normaux" ont été traités avec une statine hydrosoluble (pravastatine P, 20 à 100 µM), et avec un aminobiphosphonate (NBP zolédronate Z, 20 à 100 µM) en association (Pistes A à I, respectivement P20/Z20, P20/Z60, P20/Z100, P60/Z20, P60/Z60, P60/Z100, P100/Z20, P100/Z60, P100/Z100). Le western-blot montre "l'apparition" d'une bande correspondant à la taille de la prélamine A non mature (non tronquée) en fonction de l'augmentation de concentration des deux molécules, ce qui confirme que la farnésylation est nécessaire à la maturation de la lamine A. Ce résultat montre que le blocage de la synthèse du farnésyl-PP en 2 points de la voie métabolique est plus efficace qu'un blocage en un seul point sur l'inhibition de la farnésylation de la prélamine A, au moins *ex-vivo.*

### B.2) Dose- et durée-réponse en immunohistochimie (Figure 2)

Des courbes dose-réponse et durée-réponse ont permis de déterminer une efficacité maximale en mesurant 2 paramètres sur des cellules témoins saines d'une part, puis sur des cellules de patients HGPS. L'association pravastatine (hydrosoluble) / zolédronate (NBP) la plus efficace est obtenue pour 1 µM de pravastatine pendant 24 h, puis zolédronate pendant 12 h : sur les cellules saines, aucune toxicité n'est observée, alors que sur les cellules HGPS, (Cellules avec anomalies nucléaires), le nombre de noyaux "déformés" baisse de 75 à 40 %. Dans le même temps, le taux de prélamine A obtenu sur cellules saines est maximal.

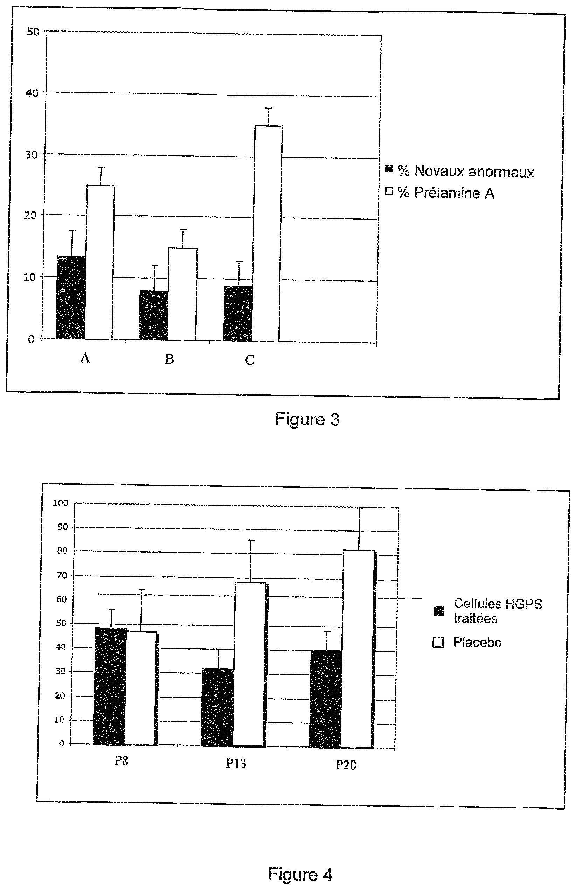

### B.3) Effet du traitement en immunohistochimie (Figure 3)

L'action combinée de la pravastatine et du zolédronate montre une meilleure efficacité, puisque le taux de prélamine A produit dans des cellules saines traitées (estimé à 35%) est beaucoup plus élevé en association que si les molécules sont ajoutées seules (respectivement 25 et 15 %). Au contraire, le taux de noyaux déformés (signe d'une toxicité sur les cellules saines) est minimal (inférieur à 10%), moindre de ce qu'il est sur des cellules traitées avec de la pravastatine seule par exemple (environ 12 %). Traitement: Pravastatine 100 µM pendant 12h, Zolédronate 20 µM pendant 6h.

### B.4) Action sur des cellules âgées en immunohistochimie (Figure 4)

En fonction du nombre de "passages" (nombre de repiquage des cellules), donc de l'âge des cellules, la proportion de noyaux anormaux augmente, ce qui est une caractéristique des cellules HGPS non traitées. Si on les traite, cette proportion se maintient, et diminue même un peu (moins de 40% contre plus de 80% chez les cellules traitées avec un placébo). Traitement: Pravastatine 1 µM pendant 24h, Zolédronate 1 µM pendant 12h.

### B.5) Conclusion

L'association pravastatine/zolédronate est efficace à des doses pour lesquelles quasiment aucun effet n'est observé avec les molécules administrées séparément.

L'effet physiologique de blocage de la voie de prénylation est donc obtenu avec des doses bien inférieures à celles utilisées en traitement unique dans des articles publiés sur des cultures de cellules (Kusuyama & al 2006, 10 µM de pravastatine seule sur progéniteurs de cellules vasculaires ; Flint & al 1997, 25 µM de pravastatine seule sur cellules de muscle de rat néonatal).

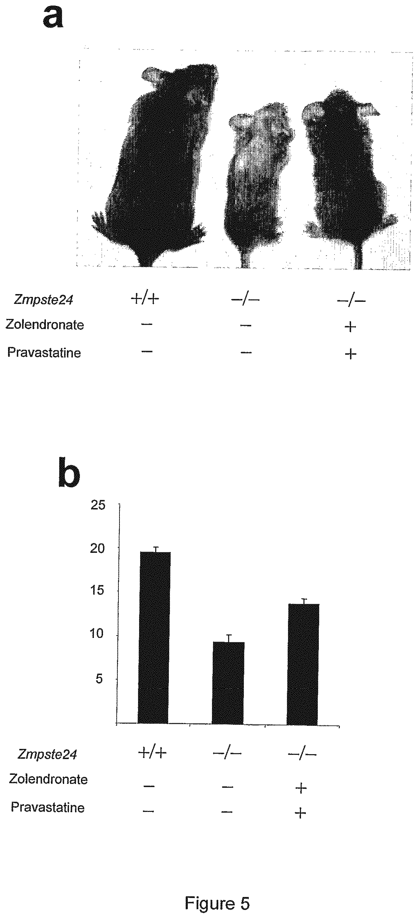

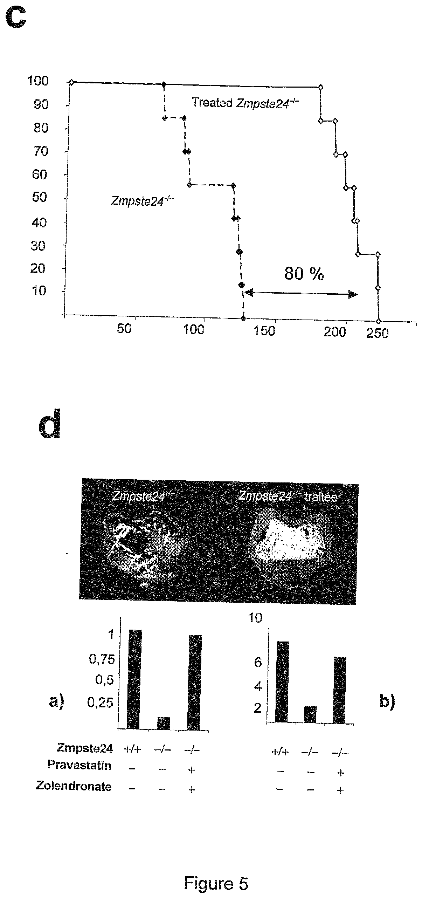

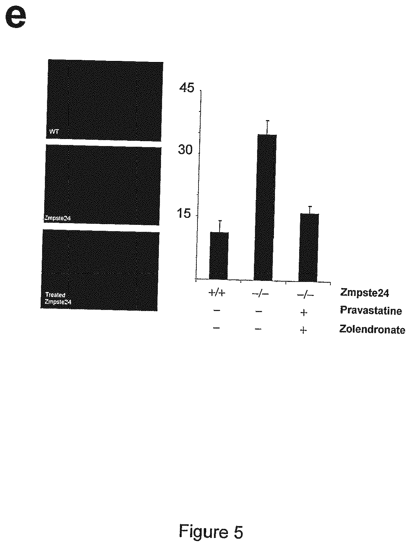

EXEMPLE 2 : Effet de l'association d'un inhibiteur de l'hydroxyméthylglutaryl-coenzyme A (HMG-CoA) réductase hydrosoluble et d'un inhibiteur de la farnésyl-pyrophosphate synthase sur un modèle de souris présentant un syndrome progéroïde (Figure 5)

Les souris KO *Zmpste24*^{*-*/*-*} utilisées ici sont celles décrites dans l'article cité de Varela & al 2005. Elles sont élevées dans une animalerie en atmosphère contrôlée, en cycle de 12 h jour / 12 h nuit, à une température de 20±2°C sous hygrométrie de 50% et avec accès à volonté à la nourriture et à l'eau. Les 2 molécules (Zometa 100 µg/kg/jour et Pravastatine 100 mg/kg/jour) sont dissoutes dans du PBS 1X et injectées par voie intrapéritonéale, quotidiennement, sur des souris âgées de 1 mois et jusqu'à leur décès. Les contrôles sont des souris sauvages de même portée, traitées au PBS 1X seul. Le poids est mesuré hebdomadairement, la survie des souris est reportée sur une courbe (Figure 5b).

A la mort des souris, les tibias sont disséqués et analysés en tomographie par scanner à µCT. Le nombre de trabécules par millimètre est mesuré par la méthode décrite dans Hildebrand & al 1997 (Figure 5d).

Les cellules du foie sont prélevées, fixées dans du paraformaldéhyde à 4°C, incluses en paraffine et analysées en microscopie confocale après marquage de la chromatine au DAPI (4'6-DiAmidino-2-Phényllndole). Le pourcentage de noyaux anormaux est estimé par comptage (Figure 5e).

La survie des souris traitées est grandement améliorée, elle est maximale notamment pour les femelles, avec un allongement de la durée de vie moyenne de 80% environ (Figure 5c). Les symptômes cliniques de la maladie sont tous considérablement réduits par rapport aux individus traités avec le PBS seul.

### Références

- Basso AD, Kirschmeier P, Bishop WR. Farnesyl transferase inhibitors. J Lipid Res 47:15-31, 2006.
- Biamonti G, Giacca M, Perini G, Contreas G, Zentilin L, Weighardt F, Guerra M, Della Valle G, Saccone S, Riva S et al. The gene for a novel human lamin maps at a highly transcribed locus of chromosome 19 which replicates at the onset of S-phase. Mol Cell Biol 12:3499-3506, 1992.
- Bishop WR, Kirschmeier P, Baun C. Farnesyl transferase inhibitors: mechanism of action, translational studies and clinical evaluation. Cancer Biol Ther 2:S96-104, 2003.
- Broers JL, Hutchinson CJ, Ramaekers FC. Laminopathies. J Pathol 204:478-488, 2004.
- Broers JLV, Ramaekers FCS, Bonne G, Ben Yaou R, Hutchinson CJ. Nuclear lamins: laminopathies and their role in premature aging. Physiol Rev 86:967-1008, 2006.
- Cadinanos J, Varela I, Lopez-Otin C, Freije JM. From immature lamin to premature aging: molecular pathways and therapeutic opportunities. Cell Cycle 4:1732-1735, 2005.
- Capell BC, Erdos MR, Madigan JP, Fiordalisi JJ, Varga R, Conneely KN, Gordon LB, Der CJ, Cox AD, Collins FS. Inhibiting farnesylation of progerin prevents the characteristic nuclear blebbing of Hutchinson-Gilford progeria syndrome. Proc Natl Acad Sci USA 102:12879-12884, 2005.
- De Sandre-Giovannoli A, Bernard R, Cau P, Navarro C, Amiel J, Boccaccio I, Lyonnet S, Stewart CL, Munnich A, Le Merrer M, Levy N. Lamin A truncation in Hutchinson-Gilford progeria. Science 300:2055, 2003.
- Demyanets S, Kaun C, Pfaffenberger S, Philipp J. Hohensinner PJ, Rega G, Pammer J, Maurer G, Huber K, Wojta J. Hydroxymethylglutaryl-coenzyme A reductase inhibitors induce apoptosis in human cardiac myocytes in vitro. Biochem Pharmacol 71:1324-1330, 2006.
- Duque G, Rivas D. Age-related changes in Lamin A/C expression in the osteoarticular system: laminopathies as a potentiel new aging mechanism. Mech Aging Dev 127:378-383, 2006.
- Efuet ET, Keyomarsi K. Farnesyl and geranylgeranyl transferase inhibitors induce G1 arrest by targeting the proteasome. Cancer Res 66:1040-1051, 2006.
- Eriksson M, Brown WT, Gordon LB, Glynn MW, Singer J, Scott L, Erdos MR, Robbins CM, Moses TY, Berglund P, Dutra A, Pak E, Durkin S, Csoka AB, Boehnke M, Glover TW, Collins FS. Recurrent de novo point mutation in lamin A cause Hutchinson-Gilford progeria syndrome. Nature 423:293-298, 2003.
- Evans M, Rees A. The myotoxicity of statins. Cur Op Lipid, 13:415-420, 2002.
- Flint OP, Masters BA, Gregg RE, Durham SK. HMG CoA reductase inhibitor-induced myotoxicity: pravastatin and lovastatin inhibit the geranylgeranylation of low-molecular-weight proteins in neonatal rat muscle ce// culture. Tox Appl Pharmacol 145:99-110, 1997.
- Fong LG, Frost D, Meta M, Qiao X, Yang SH, Coffinier C, Young SG. A protein farnesyltransferase inhibitor ameliorates disease in a mouse model of progeria. Science,311 : 1621-1623, 2006.
- Fong LG, Ng JK, Lammerding J, Vickers TA, Meta M, Coté N, Gavino B, Qiao X, Chang SY, Young SR, Yang SH, Stewart CL, Lee RT, Bennett CF, Bergo MO, Young SG. Prelamin A and Lamin A appear to be dispensable in the nuclear lamina. J Clin Invest 116:743-752, 2006.
- Fong LG, Ng JK, Meta M, Cote N, Yang SH, Stewart CL, Sullivan T, Burghardt A, Majumdar S, Reue K, Bergo MO, Young SG. Heterozygosity for Lmna deficiency eliminates the progeria-like phenotypes in Zmpste24-deficient mice. Proc Natl Acad Sci USA 101:18111-18116, 2004.
- Glynn MW, Glover TW. Incomplete processing of mutant lamin A in Hutchison-Gilford progeria leads to nuclear abnormalities, which are reversed by farnesyltransferase inhibition. Hum Mol Genet 14:2959-2969, 2005.
- Goldman RD, Shumaker DK, Erdos MR, Eriksson M, Goldman AE, Gordon LB, Gruenbaum Y, Khuon S, Mendez M, Varga R, Collins FS. Accumulation of mutant lamin A causes progressive changes in nuclear architecture in Hutchinson-Gilford progeria syndrome. Proc Natl Acad Sci USA 101:8963-8968, 2004.
- Gruenbaum Y, Margalit A, Goldman RD, Shumaker DK, Wilson KL. The nuclear lamina comes of age. Nat Mol Cell Biol 6:21-31, 2005.
- Gruenbaum Y, Wilson KL, Harel A, Goldberg M, Cohen M. Review: nuclear lamins - structural proteins with fundamental functions. J Struct Biol 129:313-323, 2000.
- Hampton R, Dimster-Denk D, Rine J. The biology of HMG-CoA reductase: the pros of contra-regulation. Trends Biochem Sci 21:140-145, 1996.
- Harborth J, Elbashir SM, Bechert K, Tuschl T, Weber K. Identification of essential genes in cultured mammalian cells using small interfering RNAs. J Cell Sci 114:4557-4565, 2001.
- Hegele RA, Cao H, Liu DM, Costain GA, Charlton-Menys V, Rodger NW, Durrington PN. Sequencing of reannotated LMNB2 gene reveals novel mutations in patients with acquired partial lipodystrophy. Am J Hum Genet 79:383-389, 2006.
- Hildebrand T, Ruegsegger P. A new method for the model independent assessment of thickness in three dimensional images. J Microsc 185:67-75, 1997.
- Hoffmann GF, Charpentier C, Mayatepek E, Mancini J, Leichsenring M, Gibson KM, Divry P, Hrebicek M, Lehnert W, Sartor K. Clinical and biochemical phenotype in 11 patients with mevalonic aciduria. Pediatrics 91:915-921, 1993.
- Huang S, Chen L, Libina N, Janes J, Martin GM, Campisi J, Oshima J. Correction of cellular phenotypes of Hutchinson-Gilford Progeria cells by RNA interference. Hum Genet. 2005 Oct 6;:1-7
- Hutchinson CJ, Worman HJ. A-type lamins: guardians of the soma? Nat Cell Biol 6:1062-1067, 2004.
- Ji JY, Lee RT, Vergnes L, Fong LG, Stewart CL, Reue K, Young SG, Zhang Q, Shanahan CM, Lammerding J. Cell nuclei spin in the absence of Lamin B1. J Biol Chem online, 8/05/2007.
- Kusuyama T, Omura T, Nishiya D, Enomoto S, Matsumoto R, Murata T, Takeuchi K, Yoshikawa J, Yoshiyama M. The effects of HMG-CoA reductase inhibitor on vascular progenitor cells. J Pharmacol Sci 1001:344-349, 2006.
- Leung KF, Baron R, Seabra MC. Geranylgeranylation of Rab GTPases. J Lipid Res 47:467-475, 2006.
- Lévy N, Cau P. Anomalies du noyau et maladies. Pour la Science 313 :2-7, 2003.
- Lin F, Worman HJ. Structural organization of the human gene (LMNB1) encoding nuclear lamin B1. Genomics 27:230-236, 1995.
- Lin F, Worman HJ. Structural organization of the human gene encoding nuclear lamin A and nuclear lamin C. J Biol Chem 268:16321-16326, 1993.
- Maraldi NM, Squarzoni S, Sabatelli P, Capanni C, Mattioli E, Ognibene A, Lattanzi G. Laminopathies: involvement of structural nuclear proteins in the pathogenesis of an increasing number of human diseases. J Cell Physiol 203:319-327, 2005.
- Mattout A, Dechat T, Adam SA, Goldman RD, Gruenbaum Y. Nuclear lamins, diseases and aging. Cur Op Cell Biol 18:335-341, 2006.
- Navarro CL, Cadinanos J, De Sandre-Giovannoli A, Bernard R, Courrier S, Boccaccio I, Boyer A, Kleijer WJ, Wagner A, Giuliano F, Beemer FA, Freije JM, Cau P, Hennekam RC, Lopez-Otin C, Badens C, Levy N. Loss of ZMPSTE24 (FACE-1) causes autosomal recessive restrictive dermopathy and accumulation of Lamin A precursors. Hum Mol Genet 14:1503-1513, 2005.
- Navarro CL, De Sandre-Giovannoli A, Bernard R, Boccaccio I, Boyer A, Genevieve D, Hadj-Rabia S, Gaudy-Marqueste C, Smitt HS, Vabres P, Faivre L, Verloes A, Van Essen T, Flori E, Hennekam R, Beemer FA, Laurent N, Le Merrer M, Cau P, Levy N. Lamin A and ZMPSTE24 (FACE-1) defects cause nuclear disorganization and identify restrictive dermopathy as a lethal neonatal laminopathy. Hum Mol Genet 13:2493-2503, 2004.
- Padiath QS, Saigoh K, Schiffmann R, Asahara H, Yamada T, Koeppen A, Hogan K, Ptacek LJ, Fu YH. Lamin B1 duplications cause autosomal dominant leukodystrophy. Nature Genet 38:1114-1123, 2006.
- Pendas AM, Zhou Z, Cadinanos J, Freije JM, Wang J, Hultenby K, Astudillo A, Wernerson A, Rodriguez F, Tryggvason K, Lopez-Otin C. Defective prelamin A processing and muscular and adipocyte alterations in Zmpste24 metalloproteinase-deficient mice. Nat Genet 31:94-99, 2002.
- Reid TS, Terry KL, Casey PJ, Beese LS. Crystallographic analysis of CaaX prenyltransferases complexed with substrates defines rules of protein substrate selectivity. J Mol Biol 343:417-433, 2004.
- Robber RA, Weber K, Osborn M. Differential timing of nuclear lamin A/C expression in the various organs of the mouse embryo and the young animal: a developmental study. Development 105:365-378, 1989.
- Scaffidi P, Misteli T. Lamin A-dependent nuclear defects in human aging. Sciencexpress, 27 avril 2006.
- Scaffidi P, Misteli T. Reversal of the cellular phenotype in the premature aging disease Hutchinson-Gilford progeria syndrome. Nat Med 11:440-445, 2005.
- Scaffidi P, Misteli T. Reversal of the cellular phenotype in the premature aging disease Hutchinson-Gilford progeria syndrome. Nature Med 11:440-445, 2005.
- Shelton KR, Egle PM, Cochran DL. Nuclear envelope proteins: identification of lamin B subtypes. Biochem Biophys Res Comm 103:975-981, 1981.
- Shumaker DK, Kuczmarski ER, Goldman RD. The nucleoskeleton: lamins an actin are major players in essential nuclear functions. Curr Op Cell Biol 15:358-366, 2003.
- Stewart C, Burke B. Teratocarcinoma stem cells and early mouse embryos contain only a single major lamin polypeptide closely resembling lamin B. Cell 51:383-392, 1987.
- Takedaa M, Noshiroa R, Onozatob ML, Tojob A, Hasannejada H, Huangc XL, Narikawac S, Endoua H. Evidence for a role of human organic anion transporters in the muscular side effects of HMG-CoA reductase inhibitors. Eur J Pharm 483 :133- 138, 2004.
- Toth JI, Yang SH, Qiao X, Beigneux AP, Gelb MH, Moulson CL, Miner JH, Young SG, Fong LG. Blocking protein farnesyltransferase improves nuclear shape in fibroblasts from humans with progeroid syndromes. Proc Natl Acad Sci USA 102:12873-12878, 2005.
- Tsai MY, Wang S, Heidinger JM, Shumaker DK, Adam SA, Goldman RD, Zheng Y. A mitotic lamin B matrix induced by RanGTP required for spindle assembly. Science 311:1887-1893, 2006.
- Varela I, Cadinanos J, Pendas AM, Gutierrez-Fernandez A, Folgueras AR, Sanchez LM, Zhou Z, Rodriguez FJ, Stewart CL, Vega JA, Tryggvason K, Freije JM, Lopez-Otin C. Accelerated ageing in mice deficient in Zmpste24 protease is linked to p53 signalling activation. Nature 437:564-568, 2005.
- Vergnes L, Peterfy M, Bergo MO, Young SG, Reue K. Lamin B1 is required for mouse development and nuclear integrity. Proc Natl Acad Sci USA 101:10428-10433, 2004.
- Winter-Vann AM, Casey PJ. Post-prenylation-processing enzymes as new targets in oncogenesis. Nat Rev Cancer 5:405-412, 2005.
- Wydner KL, McNeil JA, Lin F, Worman HJ, Lawrence JB. Chromosomal assignment of human nuclear envelope protein genes LMNA, LMNB1 and LBR by fluorescence in situ hybridization. Genomics 32:474-478, 1996.
- Young SG, Meta M, Yang SH, Fong LG. Prelamin A farnesylation and progeroid syndromes. J Biol Chem 281:39741-39745, 2006.
- Zastrow MS, Vlcek S, Wilson KL. Proteins that bind A-type lamins: integrating isolated clues. J Cell Sci 117:979-987, 2004.
- Zhang FL, Casey PJ. Protein prenylation: molecular mechanisms and functionnal consequences. Annu Rev Biochem 65:241-269, 1996.

## Revendications

1. Composition comprenant un inhibiteur de l'hydroxyméthylglutaryl-coenzyme A (HMG-CoA) réductase et d'un inhibiteur de la farnésyl-pyrophosphate synthase, ou de l'un de leurs sels physiologiquement acceptable, pour son utilisation dans le traitement des situations, pathologiques impliquant des signes de vieillissement, qu'il soit naturel, prématuré ou accéléré choisies parmi la détérioration de l'endothélium vasculaire le vieillissement de la peau, et la lyse osseuse, liées à l'accumulation et/ou la persistance dans les cellules de progérine et/ou de prélamine A farnésylée.

2. Composition pour son utilisation selon la revendications 1, dans laquelle l'inhibiteur de la farnésyl-pyrophosphate synthase est une molécule de la famille des aminobiphosphonates (NBP) ou l'un de ses sels physiologiquement acceptable.

3. Composition pour son utilisation selon la revendication 2, dans laquelle l'aminobiphosphonate peut être choisi parmi
- l'acide alendronique ou sa forme ionique, l'alendronate ;
- l'acide ibandronique, ou sa forme ionique, l'ibandronate ;
- l'acide néridronique ou sa forme ionique, le néridronate ;
- l'acide olpadronique ou sa forme ionique, l'olpadronate ;
- l'acide pamidronique ou sa forme ionique, le pamidronate ;
- l'acide risédronique ou sa forme ionique, le risédronate ;
- l'acide zolédronique (ou zolendronique) ou sa forme ionique le zolédronate (ou zolendronate) ;
- l'acide 4-N,N-diméthylaminométhane diphosphonique ou sa forme ionique le diméthylaminométhanediphosphonate ;
- l'[alpha]-amino-(4-hydroxybenzylidène) diphosphonate ;- préférentiellement l'acide zolédronique ou sa forme ionique, le zolédronate.

4. Composition pour son utilisation selon l'une quelconque des revendications précédentes, dans laquelle l'inhibiteur de HMG-CoA réductase est une molécule de la famille des statines ou l'un de ses sels physiologiquement acceptable.

5. Composition pour son utilisation selon la revendication 4, dans laquelle l'inhibiteur de HMG-CoA réductase est une statine hydrosoluble.

6. Composition pour son utilisation selon la revendication 5, dans laquelle la statine peut être choisie parmi l'atorvastatine, la simvastatine, la pravastatine, la rivastatine, la mévastatine, la fluindostatine, la vélostatine, la fluvastatine, la dalvastatine, la cérivastatine, la pentostatine, la rosuvastatine, la pitavastatine, ou la lovastatine, ou de l'un de leurs sels physiologiquement acceptable.

7. Utilisation non thérapeutique de la composition telle que définie dans l'une quelconque des revendications 1 à 6, dans le traitement de situations non pathologiques impliquant des signes de vieillissement naturel choisies parmi la détérioration de l'endothélium vasculaire et le vieillissement de la peau, liées à l'accumulation et/ou la persistance dans les cellules de progérine et/ou de prélamine A farnésylée.

8. Utilisation selon la revendication 7 dans laquelle les situations non pathologiques liées à l'accumulation et/ou la persistance dans les cellules de progérine et/ou de prélamine A farnésylée sont choisies dans le groupe comprenant le vieillissement cellulaire au niveau de la peau et/ou de l'endothélium vasculaire.

## Patentansprüche

1. Zusammensetzung, umfassend einen Hemmer der Hydroxymethylglutarylcoenzym A (HMG-CoA)-Reduktase und einen Hemmer der Farnesylpyrophosphatsynthase oder eines ihrer physiologisch annehmbaren Salze, für seine Verwendung bei der Behandlung von Erkrankungszuständen, die Anzeichen der Alterung implizieren, entweder natürlich, vorzeitig oder beschleunigt, ausgewählt aus der Verschlechterung des vaskulären Endothels, der Alterung der Haut und der Knochenlyse, verbunden mit der Ansammlung und/oder dem Fortbestehen von Progerin und/oder farnesyliertem Prolamin-A in den Zellen.

2. Zusammensetzung für ihre Verwendung nach Anspruch 1, wobei der Hemmer der Farnesylpyrophosphatsynthase ein Molekül der Familie der Aminobiphosphonate (NBP) oder eines ihrer physiologisch annehmbaren Salze ist.

3. Zusammensetzung für ihre Verwendung nach Anspruch 2, wobei das Aminobiphosphonat ausgewählt sein kann aus:
- Alendronsäure oder ihrer ionischen Form, Alendronat;
- Ibandronsäure oder ihrer ionischen Form, Ibandronat;
- Neridronsäure oder ihrer ionischen Form, Neridronat;
- Olpadronsäure oder ihrer ionischen Form, Olpadronat;
- Pamidronsäure oder ihrer ionischen Form, Pamidronat;
- Risedronsäure oder ihrer ionischen Form, Risedronat;
- Zoledron- (oder zoledronsiche) Säure oder ihrer ionischen Form, Zoledronat (oder Zolendronat);
- 4-N,N-Dimethylamino-methan-diphosphonsäure oder ihrer ionischen Form, Dimethylaminmethandiphosphonat;
- [Alpha]amino-(4-hydroxybenzyliden)diphosphonat;
- vorzugsweise Zoledronsäure oder ihrer ionischen Form, Zoledronat.

4. Zusammensetzung für ihre Verwendung nach einem beliebigen der vorhergehenden Ansprüche, wobei der Hemmer der HMG-CoA-Reduktase ein Molekül der Familie der Statine oder eines seiner physiologisch annehmbaren Salze ist.

5. Zusammensetzung für ihre Verwendung nach Anspruch 4, wobei der Hemmer der HMG-CoA-Reduktase ein wasserlösliches Statin ist.

6. Zusammensetzung für ihre Verwendung nach Anspruch 5, wobei das Statin ausgewählt sein kann aus Atorvastatin, Simvastatin, Pravastatin, Rivastatin, Mevastatin, Fluindostatin, Velostatin, Fluvastatin, Dalvastatin, Cerivastatin, Pentostatin, Rosuvastatin, Pitavastatin oder Lovastatin oder einem ihrer physiologisch annehmbaren Salze.

7. Nicht therapeutische Verwendung der Zusammensetzung wie in einem beliebigen der Ansprüche 1 oder 6 definiert, bei der Behandlung von Nicht-Erkrankungszuständen, die natürliche Anzeichen der Alterung implizieren, ausgewählt aus der Verschlechterung des vaskulären Endothels, und der Alterung der Haut, verbunden mit der Ansammlung und/oder dem Fortbestehen von Progerin und/oder farnesyliertem Prolamin-A in den Zellen.

8. Verwendung nach Anspruch 7, wobei die Nicht-Erkrankungszustände, verbunden mit der Ansammlung und/oder dem Fortbestehen von Progerin und/oder farnesyliertem Prolamin-A in den Zellen, ausgewählt sind aus der Gruppe bestehend aus der Zellalterung auf der Ebene der Haut und/oder dem vaskulären Endothel.

## Claims

1. Composition comprising an inhibitor of hydroxymethylglutaryl-coenzyme A (HMG-CoA) reductase and an inhibitor of farnesyl-pyrophosphate synthase, or one of their physiologically acceptable salts, for its use in the treatment of pathological conditions presenting signs of ageing, whether it be natural, premature or accelerated selected from the group comprising deterioration of the vascular endothelium, skin aging, and bone lysis, related to the accumulation and/or persistence of progerin and /or farnesylated prelamin A.

2. Composition for its use according to claim 1, wherein the inhibitor of farnesyl-pyrophosphate synthase is a molecule in the family of aminobisphosphonates (NBP) or one of its physiological acceptable salts.

3. Composition for its use according to claim 2, wherein the aminobisphosphonate can be selected from
- alendronic acid or its ionic form, alendronate;
- ibandronic acid or its ionic form, ibandronate;
- neridronic acid or its ionic form, neridronate;
- olpadronic acid or its ionic form, olpadronate;
- pamidronic acid or its ionic form, pamidronate;
- risedronic acid or its ionic form, risedronate;
- zoledronic (or zolendronic) acid or its ionic form, zoledronate (or zolendrate);
- 4-N,N-dimethylaminomethane diphosphonic acid or its ionic form, dimethylaminomethanediphosphonate;
- α-amino-(4-hydroxybenzylidene) diphosphonate;
preferably zoledronic acid or its ionic form, zoledronate

4. Composition for its use according to any one of preceding claims, wherein the inhibitor of HMG-CoA reductase is a molecule in the statin family or one of its physiological acceptable salts.

5. Composition for its use according to claim 4, wherein the inhibitor of HMG-CoA reductase is a hydrosoluble statin.

6. Composition for its use according to claim 4, wherein the statin can be selected from atorvastatin, simvastatin, pravastatin, rivastatin, mevastatin, fluindostatin, velostatin, fluvastatin, dalvastatin, cerivastatin, pentostatin, rosuvastatin, pitavastatin or lovastatin, or one of their physiologically acceptable salts.

7. Non-therapeutic use of the composition as defined in any one of claims 1 to 6, in the treatment of non pathologic conditions involving signs of natural ageing selected from deterioration of the vascular endothelium and skin aging, related to the accumulation and/or persistence of progerin and /or farnesylated prelamin A.

8. Use according to claim 7 wherein non pathologic conditions related to the accumulation and/or persistence of progerin and /or farnesylated prelamin A are selected from the group comprising cellular aging in the skin and/or in the vascular endothelium.
